# EUROPEAN PATENT APPLICATION

(11) **EP 4 190 324 A1**
(43) Date of publication of application: **07.06.2023**
(21) Application number: 21851394.3
(22) Date of filing: 30.07.2021
(51) Int. Cl.: A61K 31/198, A61K 9/08, A61K 31/7004, A61K 38/02, A61P 3/02, A61P 7/00, A61P 7/08

(54) **NUTRITION FORMULATION**

(30) Priority: 31.07.2020 JP 2020130618
(71) Applicant: Otsuka Pharmaceutical Factory, Inc., Naruto-shi, Tokushima 772-8601 (JP)
(72) Inventor: YAMAOKA, Ippei, Naruto-shi, Tokushima 772-8601 (JP)
(74) Representative: Hoffmann Eitle
(86) International application number: PCT/JP2021/028282
(87) International publication number: WO 2022/025231

(57) **Abstract**

Provided are a novel method for providing nutritional support while suppressing symptoms of refeeding syndrome in patients in an undernutrition state; and a nutritional formulation that can be used in the method.

Provided is a nutritional formulation for use in suppressing a symptom of refeeding syndrome, the nutritional formulation comprising a protein and/or an amino acid in a total amount of 3.5 g or less per 100 kcal.

## Description

### Technical Field

The present invention relates to a nutritional formulation and use of the nutritional formulation.

### Background Art

Refeeding syndrome is a general term for a series of metabolic complications that develop as a result of providing aggressive nutritional support for chronically undernourished patients (Non-patent Literature (NPL) 1 and NPL 2). In other words, refeeding syndrome develops due to sudden start of nutritional therapy in patients in an undernutrition state. In particular, undernourished patients who are transported to medical facilities in emergency situations are likely to develop refeeding syndrome and often have difficulty with nutritional support.

Specific symptoms of refeeding syndrome include hypophosphatemia, hypokalemia, hypomagnesemia, vitamin B₁ deficiency, and the like.

### Citation List

### Non-patent Literature

NPL 1: Hisham Mehanna and three others, "Refeeding syndrome - awareness, prevention and management," 2009, Head & Neck Oncology, 1:4
NPL 2: Stanga Z and six others, "Nutrition in clinical practice - the refeeding syndrome: illustrative cases and guidelines for prevention and treatment," 2008, Eur J Clin Nutr., 62 (6): 687-94

### Summary of Invention

### Technical Problem

The present inventors focused on the fact that the means for providing nutritional support while suppressing symptoms of refeeding syndrome in patients in an undernutrition state are currently limited. An object of the present invention is to provide a novel method for providing such nutritional support and a nutritional formulation that can be used in the method.

### Solution to Problem

The present inventors conducted extensive research to achieve the above object, and found that nutritional support can be provided while suppressing symptoms of refeeding syndrome in patients in an undernutrition state by using a nutritional formulation with a low protein and/or amino acid content. The present invention has been accomplished by conducting further research based on this finding. The present invention includes the following embodiments.
Item 1.
   A nutritional formulation for use in suppressing a symptom of refeeding syndrome, the nutritional formulation comprising a protein and/or an amino acid in a total amount of 3.5 g or less per 100 kcal.
Item 2.
   The nutritional formulation according to Item 1, for use in suppressing a symptom of hypophosphatemia.
Item 3.
   The nutritional formulation according to Item 1 or 2, for use in administration to a patient in an undernutrition state.
Item 4.
   The nutritional formulation according to any one of Items 1 to 3, which is an intravenous nutritional infusion formulation.
Item 5.
   The intravenous nutritional infusion formulation according to Item 4, further comprising a sugar.
Item 6.
   The nutritional formulation according to any one of Items 1 to 3, which is an enteral nutritional formulation.
Item 7.
   The enteral nutritional formulation according to Item 6, further comprising a saccharide.
Item 8.
   A method for providing nutritional support for a patient in an undernutrition state using a nutritional formulation, wherein the nutritional formulation comprises 0 to 3.5 g of a protein or an amino acid per 100 kcal.

### Advantageous Effects of Invention

According to the present invention, nutritional support can be provided while suppressing symptoms of refeeding syndrome in patients in an undernutrition state.

### Brief Description of Drawings

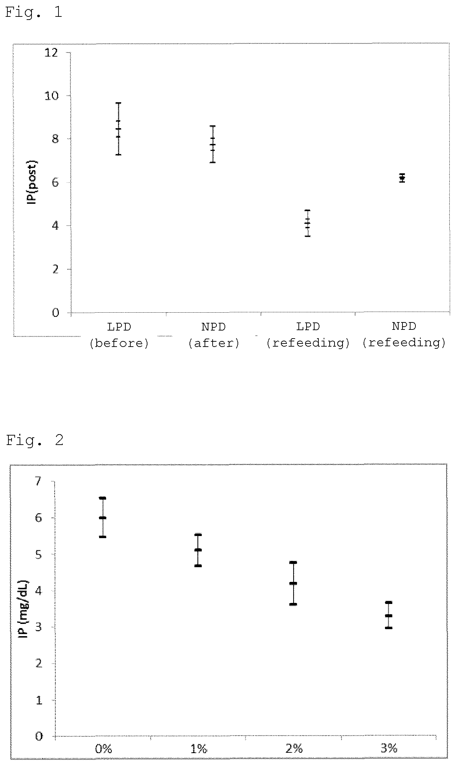

Fig. 1 shows plasma phosphorus concentrations in rats given a normal diet and rats given a low-protein diet, before and after refeeding with an intravenous nutritional infusion formulation. The unit of the plasma phosphorus concentration (vertical axis) is mg/dL. "NPD (before)" indicates the plasma phosphorus concentration before refeeding in the rats given the normal diet, and "LPD (before)" indicates the plasma phosphorus concentration before refeeding in the rats given the low-protein diet. "NPD (refeeding)" indicates the plasma phosphorus concentration after refeeding in the rats given the normal diet, and "LPD (refeeding)" indicates the plasma phosphorus concentration after refeeding in the rats given the low-protein diet.
Fig. 2 shows plasma phosphorus concentrations after refeeding in rats given a low-protein diet and refed with intravenous nutritional infusion formulations. The unit of the plasma phosphorus concentration (vertical axis) is mg/dL. "0%" indicates the infusion formulation of Example 2, "1%" indicates the infusion formulation of Example 3, "2%" indicates the infusion formulation of Example 4, and "3%" indicates the infusion formulation of Comparative Example 1.

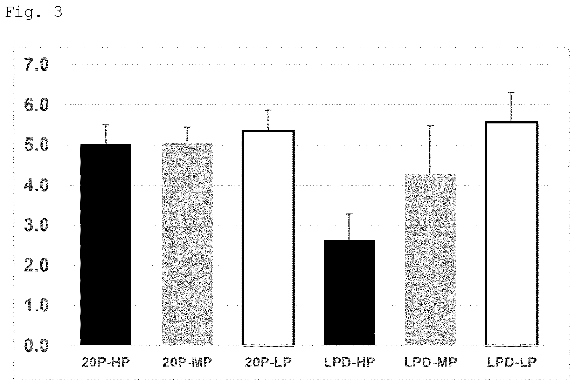

Fig. 3 shows plasma phosphorus concentrations after refeeding in rats refed with enteral nutritional formulations. The unit of the plasma phosphorus concentration (vertical axis) is mg/dL. "20P-HP" indicates a group that received a normal diet before refeeding and the liquid food of Comparative Example 2 at the time of refeeding, "20P-MP" indicates a group that received a normal diet before refeeding and the liquid food of Example 5 at the time of refeeding, and "20P-LP" indicates a group that received a normal diet before refeeding and the liquid food of Example 6 at the time of refeeding. "LDP-HP" indicates a group that received a low-protein diet before refeeding and the liquid food of Comparative Example 2 at the time of refeeding, "LDP-MP" indicates a group that received a low-protein diet before refeeding and the liquid food of Example 5 at the time of refeeding, and "LDP-LP" indicates a group that received a low-protein diet before refeeding and the liquid food of Example 6 at the time of refeeding.

### Description of Embodiments

### 1. Nutritional Formulation of Present Invention

The nutritional formulation of the present invention comprises a protein and/or an amino acid in a total amount of 3.5 g/100 kcal or less.

The nutritional formulation of the present invention is a nutritional formulation to be administered to suppress symptoms of refeeding syndrome.

Refeeding syndrome is a general term for a series of metabolic complications that develop as a result of providing aggressive nutritional support for patients in an undernutrition state. Examples of specific symptoms of refeeding syndrome include hypophosphatemia, hypokalemia, hypomagnesemia, vitamin B₁ deficiency, and the like. In particular, symptoms of hypophosphatemia can be suppressed by administering the nutritional formulation of the present invention.

The nutritional formulation of the present invention can be administered to patients in an undernutrition state who may develop refeeding syndrome.

Such patients are, for example, patients who meet one or more of the following criteria (a) to (d) prescribed in the NICE guidelines:
(a) BMI of less than 16 kg/m²
(b) unintentional weight loss of greater than 15% within the last 3 to 6 months
(c) no nutritional intake for more than 10 days
(d) hypokalemia, hypophosphatemia, hypomagnesemia before refeeding; or
   patients who meet two or more of the following criteria (e) to (h) :
(e) BMI of less than 18.5 kg/m²
(f) unintentional weight loss of greater than 10% within the last 3 to 6 months
(g) no nutritional intake for more than 5 days
(h) a history of alcohol abuse or drugs including insulin, chemotherapy, antacids, or diuretics.

Patients in an undernutrition state can be classified into the following types: the marasmus type, in which both protein and calorific value are deficient; the kwashiorkor type, in which protein deficiency is more severe than calorific value deficiency; and the marasmus-kwashiorkor type, which is an intermediate type between the two. The nutritional formulation of the present invention can effectively suppress symptoms of refeeding syndrome, especially when administered to marasmus-kwashiorkor-type undernourished patients and to kwashiorkor-type undernourished patients.

Since the protein and/or amino acid content is within the above range in the nutritional formulation of the present invention, nutritional support can be provided for patients in an undernutrition state while suppressing symptoms of refeeding syndrome.

In this respect, it is preferred that the intravenous nutritional infusion formulation of the present invention comprises a protein and/or an amino acid in a total amount of 3.5 g or less, 3.0 g or less, 2.5 g or less, 2.4 g or less, 2.0 g or less, 1.5 g or less, 1.2 g or less, or 1.0 g or less, per 100 kcal.

The nutritional formulation of the present invention may not comprise a protein and/or an amino acid, but may comprise a protein and/or an amino acid to provide sufficient nutritional support. When the nutritional formulation of the present invention comprises a protein and/or an amino acid, the total amount of protein and/or amino acid is preferably 0.1 g or more, 0.2 g or more, 0.3 g or more, 0.4 g or more, or 0.5 g or more, per 100 kcal.

In these respects, it is preferred that the intravenous nutritional infusion formulation of the present invention comprise a protein and/or an amino acid in a total amount of 0 to 3.5 g, 0 to 3.0 g, 0 to 2.5 g, 0 to 2.4 g, 0 to 2.0 g, 0 to 1.5 g, 0 to 1.2 g, 0 to 1.0 g, 0.1 to 3.5 g, 0.1 to 3.0 g, 0.1 to 2.5 g, 0.1 to 2.4 g, 0.1 to 2.0 g, 0.1 to 1.5 g, 0.1 to 1.2 g, 0.1 to 1.0 g, 0.2 to 3.5 g, 0.2 to 3.0 g, 0.2 to 2.5 g, 0.2 to 2.4 g, 0.2 to 2.0 g, 0.2 to 1.5 g, 0.2 to 1.2 g, 0.2 to 1.0 g, 0.3 to 3.5 g, 0.3 to 3.0 g, 0.3 to 2.5 g, 0.3 to 2.4 g, 0.3 to 2.0 g, 0.3 to 1.5 g, 0.3 to 1.2 g, 0.3 to 1.0 g, 0.4 to 3.5 g, 0.4 to 3.0 g, 0.4 to 2.5 g, 0.4 to 2.4 g, 0.4 to 2.0 g, 0.4 to 1.5 g, 0.4 to 1.2 g, 0.4 to 1.0 g, 0.5 to 3.5 g, 0.5 to 3.0 g, 0.5 to 2.5 g, 0.5 to 2.4 g, 0.5 to 2.0 g, 0.5 to 1.5 g, 0.5 to 1.2 g, or 0.5 to 1.0 g, per 100 kcal.

Examples of the nutritional formulation of the present invention include an intravenous infusion formulation and an enteral nutritional formulation.

### 2. Intravenous Nutritional Infusion Formulation of Present Invention

The nutritional formulation of the present invention may be, for example, an intravenous infusion formulation.

### 2.1 Amino Acid

The amino acids that can be used in the intravenous nutritional infusion formulation of the present invention are typically used in the form of a free amino acid. However, amino acids in the form of a pharmaceutically acceptable salt, an ester, an N-acyl derivative, or a dipeptide may also be used. Specific examples of free amino acids that can be incorporated in the intravenous nutritional infusion formulation of the present invention include L-leucine, L-isoleucine, L-valine, L-lysine, L-threonine, L-tryptophan, L-methionine, L-phenylalanine, L-cysteine, L-tyrosine, L-arginine, L-histidine, L-alanine, L-proline, L-serine, glycine, L-aspartic acid, L-glutamic acid, and the like. Specific examples of amino acid salts include inorganic acid salts such as L-arginine hydrochloride, L-cysteine hydrochloride, L-glutamic acid hydrochloride, L-histidine hydrochloride, and L-lysine hydrochloride; organic acid salts such as L-lysine acetate and L-lysine malate; and the like. Specific examples of amino acid esters include L-tyrosine methyl ester, L-methionine methyl ester, L-methionine ethyl ester, and the like. Specific examples of N-acyl amino acids include N-acetyl-L-cysteine, N-acetyl-L-tryptophan, N-acetyl-L-proline, and the like. Specific examples of amino acid dipeptides include L-tyrosyl-L-tyrosine, L-alanyl-L-tyrosine, L-arginyl-L-tyrosine, L-tyrosyl-L-arginine, and the like. In particular, L-cysteine is preferably incorporated in the form of acetylcysteine, in view of stability. The intravenous nutritional infusion formulation of the present invention preferably comprises at least seven types, more preferably at least eight types, and even more preferably all nine types of the essential amino acids (i.e., nine types of amino acids: L-leucine, L-isoleucine, L-valine, L-lysine, L-threonine, L-tryptophan, L-methionine, L-phenylalanine, and L-histidine).

The total amino acid concentration in the entire intravenous nutritional infusion formulation (in the case in which the infusion formulation is composed of infusions contained in a multiple-chamber container that are mixed before use, the mixture of the infusions) is, for example, 30 g/L or less, preferably 20 g/L or less, and more preferably 10 g/L or less. The intravenous infusion formulation of the present invention may not comprise an amino acid, but may comprise one or more amino acids in a total concentration of 0.1 g/L or more, preferably 1.0 g/L or more, and more preferably 3.0 g/L or more, in order to provide sufficient nutritional support.

The concentration of each amino acid in the entire intravenous nutritional infusion formulation (in the case in which the infusion formulation is composed of infusions contained in a multiple-chamber container that are mixed before use, the mixture of the infusions) is as follows: in terms of free amino acids, L-leucine: 9 g/L or less; L-isoleucine: 4.5 g/L or less; L-valine: 4.5 g/L or less; L-lysine: 5 g/L or less; L-threonine: 3 g/L or less; L-tryptophan: 1.5 g/L or less; L-methionine: 2.4 g/L or less; L-phenylalanine: 4.5 g/L or less; L-cysteine: 0.9 g/L or less; L-tyrosine: 0.6 g/L or less; L-arginine: 5 g/L or less; L-histidine: 3 g/L or less; L-alanine: 4.5 g/L or less; L-proline: 3 g/L or less; L-serine: 2.1 g/L or less; glycine: 3 g/L or less; L-aspartic acid: 0.9 g/L or less; and L-glutamic acid: 0.9 g/L or less.

### 2.2 Sugar

The intravenous nutritional infusion formulation of the present invention preferably comprises a sugar. Examples of sugars that can be incorporated include reducing sugars such as glucose, fructose, and maltose; non-reducing sugars such as xylitol, sorbitol, and glycerol; and the like. These sugars may be used singly or in a combination of two or more.

The sugar concentration in the entire intravenous nutritional infusion formulation (in the case in which the infusion formulation is composed of infusions contained in a multiple-chamber container that are mixed before use, the mixture of the infusions) is preferably 50 to 300 g/L, and more preferably 50 to 250 g/L.

When the intravenous nutritional infusion formulation of the present invention comprises an amino acid, it is preferred that the sugar and the amino acid are contained in different chambers. Examples of such a case include an embodiment in which in an intercommunicable multiple-chamber container including at least a first chamber and a second chamber isolated from each other, the first chamber contains the sugar, and the second chamber contains the amino acid.

### 2.3 Electrolyte

The intravenous nutritional infusion formulation of the present invention may further comprise an electrolyte. The electrolyte is an electrolyte that is used in the infusion field and serves as an active ingredient rather than an additive or the like. Specifically, the electrolyte is one contained in a body fluid (e.g., blood or intracellular fluid) (body fluid electrolyte), and can be referred to as a "physiologically important electrolyte." Specific examples of such electrolytes include potassium, calcium, sodium, magnesium, phosphorus, zinc, chlorine, and the like.

Examples of calcium sources include calcium salts such as calcium gluconate, calcium chloride, calcium glycerophosphate, calcium lactate, calcium pantothenate, and calcium acetate. Calcium salts may be in the form of a hydrate (e.g., calcium gluconate hydrate). The concentration of calcium in the entire intravenous nutritional infusion formulation of the present invention (in the case in which the infusion formulation is composed of infusions contained in a multiple-chamber container that are mixed before use, the mixture of the infusions) is 1 mEq/L or more, preferably 1 to 9 mEq/L, and more preferably 3 to 6 mEq/L.

Examples of potassium sources include potassium chloride, potassium acetate, potassium citrate, potassium glycerophosphate, potassium sulfate, potassium lactate, and the like. Among these, potassium glycerophosphate is preferable because it also acts as a phosphorus source. These potassium sources may be in the form of a hydrate. The concentration of potassium in the entire intravenous nutritional infusion formulation of the present invention (in the case in which the infusion formulation is composed of infusions contained in a multiple-chamber container that are mixed before use, the mixture of the infusions) is preferably 16 mEq/L or more, and more preferably 16 to 30 mEq/L.

Examples of sodium sources include sodium salts such as sodium chloride, sodium lactate, sodium acetate, sodium sulfate, sodium glycerophosphate, sodium citrate, and sodium lactate. When the infusion formulation of the present invention contains phosphorus, and calcium and/or magnesium, it is preferable to use sodium citrate as (part of) the sodium sources in order to prevent precipitation of these elements. The concentration of sodium in the entire intravenous nutritional infusion formulation of the present invention (in the case in which the infusion formulation is composed of infusions contained in a multiple-chamber container that are mixed before use, the mixture of the infusions) is, for example, 25 to 100 mEq/L, and preferably 30 to 70 mEq/L.

Examples of magnesium sources include magnesium sulfate, magnesium chloride, magnesium acetate, and the like. Magnesium sources may be in the form of a hydrate. The concentration of magnesium in the entire intravenous nutritional infusion formulation of the present invention (in the case in which the infusion formulation is composed of infusions contained in a multiple-chamber container that are mixed before use, the mixture of the infusions) is preferably 0.5 to 10 mEq/L, and more preferably 2 to 6 mEq/L.

Preferred examples of phosphorus sources are organic salts such as sodium glycerophosphate and potassium glycerophosphate, since the use of an inorganic salt as a phosphorus source may result in precipitation of calcium phosphate or magnesium phosphate. When lecithin is used as an emulsifying agent, the lecithin also acts as a phosphorus source. The concentration of phosphorus in the entire intravenous nutritional infusion formulation of the present invention (in the case in which the infusion formulation is composed of infusions contained in a multiple-chamber container that are mixed before use, the mixture of the infusions) is preferably 1 to 20 mmol/L, and more preferably 3 to 10 mmol/L.

Examples of zinc sources include zinc sulfate, zinc chloride, and the like. Zinc sources may be in the form of a hydrate. The concentration of zinc in the entire intravenous nutritional infusion formulation of the present invention (in the case in which the infusion formulation is composed of infusions contained in a multiple-chamber container that are mixed before use, the mixture of the infusions) is preferably 1.5 to 50 µmol/L.

Examples of chlorine sources include sodium chloride, potassium chloride, magnesium chloride, calcium chloride, and the like. The concentration of chlorine in the entire intravenous nutritional infusion formulation of the present invention (in the case in which the infusion formulation is composed of infusions contained in a multiple-chamber container that are mixed before use, the mixture of the infusions) is preferably 25 to 80 mEq/L, and more preferably 30 to 60 mEq/L.

### 2.4 Vitamin

Various types of vitamins can be added to the intravenous nutritional infusion formulation of the present invention. Vitamins are classified into water-soluble vitamins and fat-soluble vitamins.

Examples of water-soluble vitamins that can be added to the intravenous nutritional infusion formulation of the present invention include B vitamins and vitamin C. Examples of B vitamins include vitamin B₁ (thiamine), vitamin B₂ (riboflavin), vitamin B₃ (niacin), vitamin B₅ (pantothenic acid), vitamin B₆, vitamin B₇ (biotin), vitamin B₉ (folic acid), vitamin B₁₂ (cyanocobalamin), and the like. Examples of fat-soluble vitamins include vitamin A, vitamin D (in particular, cholecalciferol), vitamin E, vitamin K, and the like.

The concentration of vitamin C in the entire intravenous nutritional infusion formulation of the present invention (in the case in which the infusion formulation is composed of infusions contained in a multiple-chamber container that are mixed before use, the mixture of the infusions) is, for example, 25 to 250 mg/L, preferably 50 to 200 mg/L, and more preferably 40 to 100 mg/L.

Usable vitamin B₁ includes thiamine, thiamine hydrochloride, and the like. The concentration of vitamin B₁ in the entire intravenous nutritional infusion formulation of the present invention (in the case in which the infusion formulation is composed of infusions contained in a multiple-chamber container that are mixed before use, the mixture of the infusions) is, for example, 0.5 to 10 mg/L, and preferably 1 to 5 mg/L, on a thiamine basis.

Usable vitamin B₂ includes riboflavin, riboflavin sodium phosphate, flavin mononucleotide, and the like. The concentration of vitamin B₂ in the entire intravenous nutritional infusion formulation of the present invention (in the case in which the infusion formulation is composed of infusions contained in a multiple-chamber container that are mixed before use, the mixture of the infusions) is, for example, 0.5 to 10 mg/L, and preferably 0.5 to 3 mg/L, on a riboflavin basis.

Usable vitamin B₆ includes pyridoxine, and salts of pyridoxine such as pyridoxine hydrochloride. The concentration of vitamin B₆ in the entire intravenous nutritional infusion formulation of the present invention (in the case in which the infusion formulation is composed of infusions contained in a multiple-chamber container that are mixed before use, the mixture of the infusions) is, for example, 1 to 10 mg/L, and preferably 1.5 to 4.5 mg/L, on a pyridoxine basis.

The concentration of folic acid in the entire intravenous nutritional infusion formulation of the present invention (in the case in which the infusion formulation is composed of infusions contained in a multiple-chamber container that are mixed before use, the mixture of the infusions) is, for example, 0.1 to 0.7 mg/L, and preferably 0.2 to 0.4 mg/L.

Usable vitamin B₁₂ includes cyanocobalamin, hydroxocobalamin acetate, methylcobalamin, and the like. The concentration of vitamin B₁₂ in the entire intravenous nutritional infusion formulation of the present invention (in the case in which the infusion formulation is composed of infusions contained in a multiple-chamber container that are mixed before use, the mixture of the infusions) is, for example, 0.5 to 10 µg/L, and preferably 0.5 to 3 µg/L.

As niacin, for example, nicotinamide is preferably used. The concentration of niacin in the entire intravenous nutritional infusion formulation of the present invention (in the case in which the infusion formulation is composed of infusions contained in a multiple-chamber container that are mixed before use, the mixture of the infusions) is, for example, 5 to 50 mg/L, and preferably 5 to 25 mg/L.

As pantothenic acid, panthenol is preferably used. The concentration of panthenol in the entire intravenous nutritional infusion formulation of the present invention (in the case in which the infusion formulation is composed of infusions contained in a multiple-chamber container that are mixed before use, the mixture of the infusions) is, for example, 2.5 to 15 mg/L, and preferably 5 to 10 mg/L.

The concentration of biotin in the entire intravenous nutritional infusion formulation of the present invention (in the case in which the infusion formulation is composed of infusions contained in a multiple-chamber container that are mixed before use, the mixture of the infusions) is, for example, 1 to 50 µg/L, and preferably 10 to 40 µg/L.

As vitamin A, retinol palmitate is preferably used. Further, vitamin A oil formed by dissolving retinol palmitate in oil can also be used. The concentration of vitamin A in the entire intravenous nutritional infusion formulation of the present invention (in the case in which the infusion formulation is composed of infusions contained in a multiple-chamber container that are mixed before use, the mixture of the infusions) is, for example, 500 to 2500 IU/L, and preferably 1000 to 2000 IU/L. "IU" stands for international unit. It is also called vitamin A unit.

As vitamin D, cholecalciferol (vitamin D₃) is preferably used. The concentration of vitamin D in the entire intravenous nutritional infusion formulation of the present invention (in the case in which the infusion formulation is composed of infusions contained in a multiple-chamber container that are mixed before use, the mixture of the infusions) is, for example, 0.5 to 10 µg/L, and preferably 0.5 to 3 µg/L.

As vitamin E, tocopherol acetate is preferably used. The concentration of vitamin E in the entire intravenous nutritional infusion formulation of the present invention (in the case in which the infusion formulation is composed of infusions contained in a multiple-chamber container that are mixed before use, the mixture of the infusions) is, for example, 1 to 25 mg/L, and preferably 2.5 to 10 mg/L.

As vitamin K, phytonadione (vitamin K₁) is preferably used. The concentration of vitamin K in the entire intravenous nutritional infusion formulation of the present invention (in the case in which the infusion formulation is composed of infusions contained in a multiple-chamber container that are mixed before use, the mixture of the infusions) is, for example, 20 to 1200 µg/L, and preferably 30 to 1000 µg/L.

### 2.7 Other components

Further, various known additives that can be added to intravenous nutritional infusion formulations may be also optionally incorporated. Examples of such additives include pH adjusters. The pH adjusters may be those that are known to be used in infusion formulations. For example, organic acids and amino acids may be used, in addition to acids such as hydrochloric acid, and alkalis such as sodium hydroxide and potassium hydroxide. Examples of organic acids include acetic acid, lactic acid, and the like. Examples of amino acids include L-lysine and the like. Among these, oil-soluble materials may be mixed in advance with oily components of the fat emulsion, while water-soluble materials may be mixed with water for injection, or may be added to the aqueous phase of the obtained fat emulsion. The amounts of additives can be suitably determined, and may be, for example, the same as conventionally known amounts.

Further, the intravenous nutritional infusion formulation of the present invention may optionally comprise a stabilizer. Examples of stabilizers include sulfurous acid salts such as sodium bisulfite.

As a solvent in the infusion, distilled water for injection can be typically used.

### 2.8 Container

The intravenous nutritional infusion formulation of the present invention may be an infusion contained in a single container or may be infusions contained in a multiple-chamber container that are mixed before use.

The multiple-chamber container for containing infusions may be any container that has multiple chambers that are intercommunicable. Examples include multiple-chamber containers (infusion bags) in which the chambers are separated by a partition wall that can be communicably opened, such as ones in which a partition wall is formed by an easily peelable seal (Japanese Unexamined Patent Application Publication No. H2-4671, Japanese Unexamined Utility Model Application Publication No. H5-5138, and the like), ones in which a partition wall is formed by clipping the space between the chambers (Japanese Unexamined Patent Application Publication No. S63-309263 and the like), and ones in which various communicating means that can open the partition wall is provided to the partition wall (Japanese Examined Patent Publication No. S63-20550 and the like).

Further, various plastics commonly used for medical containers etc. can be used as materials of the container for containing the intravenous nutritional infusion formulation of the present invention. Examples include flexible plastics, such as polyethylene, polypropylene, polyvinyl chloride, crosslinked ethylene-vinyl acetate copolymer, ethylene-α-olefin copolymer, blends of such polymers, and laminates comprising such polymers.

### 2.9 Administration Method

The administration method for the intravenous nutritional infusion formulation of the present invention is not particularly limited, and peripheral parenteral nutrition (PPN) and total parenteral nutrition (TPN) methods can be used. The dose of the intravenous nutritional infusion formulation of the present invention may be, for example, 100 to 3000 mL per day. This allows nutritional support to be provided for patients in an undernutrition state while suppressing symptoms of refeeding syndrome. In particular, to suppress symptoms of refeeding syndrome, it is preferred that the dose is started at 10 kcal/kg/day and gradually increased over a period of 4 to 7 days. For extremely undernourished patients with a BMI of less than 14 kg/m², it is preferred that the dose is started at 5 kcal/kg/day. Thus, refeeding syndrome can be prevented by using the intravenous nutritional infusion formulation of the present invention.

### 3. Enteral Nutritional Formulation of Present Invention

The nutritional formulation of the present invention may be, for example, an enteral nutritional formulation.

### 3.1 Protein and Amino Acid

Examples of the origins of proteins and/or amino acids that can be used in the enteral nutritional formulation of the present invention include soybean-derived peptides, collagen peptides, casein (including casein peptides obtained by hydrolysis of casein), whey protein (including whey peptides obtained by hydrolysis of whey protein), free amino acids, and the like, with whey protein being preferable.

### 3.2 Saccharide

The enteral nutritional formulation of the present invention may comprise a saccharide. In the present invention, "saccharide" refers to a carbohydrate that is degraded by a digestive enzyme and used as an energy source. The type of saccharide used in the present invention is not particularly limited. Examples of saccharides include monosaccharides such as glucose, galactose, fructose, and xylose; disaccharides such as sucrose, lactose, and maltose; oligosaccharides such as galactooligosaccharides, xylooligosaccharides, soybean oligosaccharides, fructooligosaccharides, and lactosucrose; polysaccharides such as dextrin, maltodextrin, and starch; and the like. These saccharides may be used singly or in a combination of two or more.

The amount of saccharide in the enteral nutritional formulation of the present invention may be, for example, 8 to 20 g/100 kcal, and preferably 8 to 18 g/100 kcal.

### 3.3 Lipid

The enteral nutritional formulation of the present invention may comprise a lipid. The term "lipid" includes fatty acids. The amount of lipid in the enteral nutritional formulation of the present invention may be, for example, 1 to 12 g/100 kcal, and preferably 1 to 9 g/100 kcal.

The enteral nutritional formulation of the present invention may comprise, for example, an unsaturated fatty acid and a saturated fatty acid.

As unsaturated fatty acids, either a monounsaturated fatty acid or a polyunsaturated fatty acid, or both can be incorporated.

The number of carbon atoms of fatty acids that can be incorporated into the enteral nutritional formulation of the present invention is not particularly limited. For example, a C₂₋₃₀ fatty acid can be incorporated.

Examples of saturated fatty acids that can be incorporated into the enteral nutritional formulation of the present invention include acetic acid, butyric acid, caproic acid, caprylic acid, capric acid, lauric acid, myristic acid, palmitic acid, stearic acid, arachidic acid, behenic acid, lignoceric acid, and the like. The enteral nutritional formulation of the present invention may comprise one or more of these.

Examples of monounsaturated fatty acids that can be incorporated into the enteral nutritional formulation of the present invention include palmitoleic acid, oleic acid, elaidic acid, vaccenic acid, erucic acid, and the like. The enteral nutritional formulation of the present invention may comprise one or more of these.

Examples of polyunsaturated fatty acids that can be incorporated into the enteral nutritional formulation of the present invention include linoleic acid, γ-linolenic acid, α-linolenic acid, dihomo-γ-linolenic acid, arachidonic acid, eicosapentaenoic acid, docosahexaenoic acid, and the like. The enteral nutritional formulation of the present invention may comprise one or more of these.

The enteral nutritional formulation of the present invention may comprise cholesterol.

### 3.4 Dietary Fiber

The enteral nutritional formulation of the present invention may comprise a dietary fiber. The amount of dietary fiber in the enteral nutritional formulation of the present invention may be, for example, 0.5 to 2.0 g/100 kcal, and preferably 0.6 to 1.6 g/100 kcal.

The type of dietary fiber is not particularly limited. Examples of dietary fibers include pectin, alginic acid, salts of alginic acid (e.g., sodium salt), gellan gum, carrageenan, gum ghatti, gum arabic, karaya gum, gum tragacanth, locust bean gum, resistant starch, konjac mannan, glucomannan, β-glucan, indigestible dextrin, polydextrose, inulin, indigestible oligosaccharides, agarose, fucoidan, porphyran, laminaran, guar gum, and the like.

### 3.5 Vitamin

Various types of vitamins can be added to the enteral nutritional formulation of the present invention. Vitamins are classified into water-soluble vitamins and fat-soluble vitamins.

Examples of water-soluble vitamins that can be added to the enteral nutritional formulation of the present invention include B vitamins and vitamin C. Examples of B vitamins include vitamin B₁ (thiamine), vitamin B₂ (riboflavin), vitamin B₃ (niacin), vitamin B₅ (pantothenic acid), vitamin B₆, vitamin B₇ (biotin), vitamin B₉ (folic acid), vitamin B₁₂ (cyanocobalamin), and the like. Examples of fat-soluble vitamins include vitamin A, vitamin D (in particular, cholecalciferol), vitamin E, vitamin K, and the like.

The amount of vitamin C in the enteral nutritional formulation of the present invention may be, for example, 20 to 160 mg/100 kcal, preferably 30 to 140 mg/100 kcal, and more preferably 40 to 120 mg/100 kcal.

Usable vitamin B₁ includes thiamine, thiamine hydrochloride, and the like. The amount of vitamin B₁ in the enteral nutritional formulation of the present invention may be, for example, 0.1 to 5.0 mg/100 kcal, and preferably 0.2 to 3.0 mg/100 kcal, on a thiamine basis.

Usable vitamin B₂ includes riboflavin, riboflavin sodium phosphate, flavin mononucleotide, and the like. The amount of vitamin B₂ in the enteral nutritional formulation of the present invention may be, for example, 0.1 to 0.6 mg/100 kcal, and preferably 0.2 to 0.5 mg/100 kcal, on a riboflavin basis.

Usable vitamin B₆ includes pyridoxine, and salts of pyridoxine such as pyridoxine hydrochloride. The amount of vitamin B₆ in the enteral nutritional formulation of the present invention may be, for example, 0.1 to 1.0 mg/100 kcal, and preferably 0.2 to 0.8 mg/100 kcal, on a pyridoxine basis.

The amount of folic acid in the enteral nutritional formulation of the present invention may be, for example, 10 to 120 pg/100 kcal, and preferably 20 to 100 pg/100 kcal.

Usable vitamin B₁₂ include cyanocobalamin, hydroxocobalamin acetate, methylcobalamin, and the like. The amount of vitamin B₁₂ in the enteral nutritional formulation of the present invention may be, for example, 0.1 to 1.2 pg/100 kcal, and preferably 0.2 to 1.0 µg/100 kcal.

As niacin, for example, nicotinamide is preferably used. The amount of niacin in the enteral nutritional formulation of the present invention may be, for example, 1.0 to 12 mgNE/100 kcal, and preferably 2.0 to 10 mgNE/100 kcal.

As pantothenic acid, panthenol is preferably used. The amount of panthenol in the enteral nutritional formulation of the present invention may be, for example, 0.1 to 8.0 mg/100 kcal, and preferably 0.5 to 4.0 mg/100 kcal.

The amount of biotin in the enteral nutritional formulation of the present invention may be, for example, 1.0 to 15 pg/100 kcal, and preferably 2.0 to 12 pg/100 kcal.

As vitamin A, retinol palmitate is preferably used. Further, vitamin A oil formed by dissolving retinol palmitate in oil can also be used. The amount of vitamin A in the enteral nutritional formulation of the present invention may be, for example, 20 to 200 µgRE/100 kcal, and preferably 40 to 120 pgRE/100 kcal.

As vitamin D, cholecalciferol (vitamin D₃) is preferably used. The amount of vitamin D in the enteral nutritional formulation of the present invention may be, for example, 0.1 to 8.0 pg/100 kcal, and preferably 0.5 to 4.0 pg/100 kcal.

As vitamin E, tocopherol acetate is preferably used. The amount of vitamin E in the enteral nutritional formulation of the present invention may be, for example, 1.0 to 12 mg/100 kcal, and preferably 2.0 to 10 mg/100 kcal.

As vitamin K, phytonadione (vitamin K₁) is preferably used. The amount of vitamin K in the enteral nutritional formulation of the present invention may be, for example, 1.0 to 30 µg/100 kcal, and preferably 5.0 to 20 µg/100 kcal.

### 3.6 Mineral

The enteral nutritional formulation of the present invention may comprise a mineral. Minerals that are typically used in enteral nutritional formulations can be suitably selected. Specific examples of minerals include sodium, chlorine, potassium, calcium, magnesium, phosphorus, iron, zinc, copper, manganese, iodine, selenium, chromium, molybdenum, and the like. The enteral nutritional formulation of the present invention may comprise more than one of these.

The amount of sodium in the enteral nutritional formulation of the present invention may be, for example, 10 to 600 mg/100 kcal, and preferably 50 to 400 mg.

The amount of chlorine in the enteral nutritional formulation of the present invention may be, for example, 10 to 600 mg/100 kcal, and preferably 50 to 400 mg.

The amount of potassium in the enteral nutritional formulation of the present invention may be, for example, 10 to 600 mg/100 kcal, and preferably 50 to 400 mg.

The amount of calcium in the enteral nutritional formulation of the present invention may be, for example, 10 to 400 mg/100 kcal, and preferably 20 to 200 mg.

The amount of magnesium in the enteral nutritional formulation of the present invention may be, for example, 5.0 to 200 mg/100 kcal, and preferably 10 to 100 mg.

The amount of phosphorus in the enteral nutritional formulation of the present invention may be, for example, 10 to 400 mg/100 kcal, and preferably 20 to 200 mg.

The amount of iron in the enteral nutritional formulation of the present invention may be, for example, 0.1 to 4.0 mg/100 kcal, and preferably 0.2 to 2.0 mg.

The amount of zinc in the enteral nutritional formulation of the present invention may be, for example, 0.1 to 6.0 mg/100 kcal, and preferably 0.2 to 4.0 mg.

The amount of copper in the enteral nutritional formulation of the present invention may be, for example, 0.01 to 2.0 mg/100 kcal, and preferably 0.02 to 1.0 mg.

The amount of manganese in the enteral nutritional formulation of the present invention may be, for example, 0.1 to 2.0 mg/100 kcal, and preferably 0.2 to 1.0 mg.

The amount of iodine in the enteral nutritional formulation of the present invention may be, for example, 1.0 to 60 pg/100 kcal, and preferably 5.0 to 40 µg.

The amount of selenium in the enteral nutritional formulation of the present invention may be, for example, 0.5 to 20 pg/100 kcal, and preferably 1.0 to 10 µg.

The amount of chromium in the enteral nutritional formulation of the present invention may be, for example, 0.5 to 20 pg/100 kcal, and preferably 1.0 to 10 µg.

The amount of molybdenum in the enteral nutritional formulation of the present invention may be, for example, 0.5 to 20 pg/100 kcal, and preferably 1.0 to 10 µg.

### 3.7 Other Components and Dosage Form

The amount of water in the enteral nutritional formulation of the present invention may be, for example, 30 to 150 g/100 kcal, and preferably 40 to 120 g/100 kcal.

The enteral nutritional formulation of the present invention may have a calorific concentration of, for example, 60 to 170 kcal/100 mL, and preferably 70 to 150 kcal/100 mL.

The enteral nutritional formulation of the present invention is preferably a liquid food since it is easy to ingest. However, the enteral nutritional formulation of the present invention is not limited thereto.

### 3.8 Method of Usage

The enteral nutritional formulation of the present invention is used to provide nutritional support for chronically undernourished patients. Although there is no particular limitation, for example, the enteral nutritional formulation of the present invention can be administered at preferably 100 to 2000 kcal, and more preferably 200 to 1600 kcal, per day. In particular, to suppress symptoms of refeeding syndrome, it is preferred that the dose is started at 10 kcal/kg/day and gradually increased over a period of 4 to 7 days. For extremely undernourished patients with a BMI of less than 14 kg/m², it is preferred that the dose is started at 5 kcal/kg/day. This allows nutritional support to be provided for patients in an undernutrition state while suppressing symptoms of refeeding syndrome. Thus, refeeding syndrome can be prevented by using the enteral nutritional formulation of the present invention.

### Examples

The present invention is described below with reference to Examples; however, the present invention is not limited to these Examples and the like.

### Intravenous Nutritional Infusion Formulation

### 1. Confirmation of Onset of Hypophosphatemia by Refeeding Rat in Undernutrition State

To confirm the onset of hypophosphatemia by refeeding rats in an undernutrition state, the following experiment was performed.

The animals used were male Wistar rats (7 weeks old at the time of purchase, Charles River Laboratories Japan, Inc.). After an acclimation period, a group given a purified diet having a protein content of 2.5% (hereinafter referred to as "low-protein diet") for at least 3 weeks, and a group given a purified diet having a protein content of 20% (hereinafter referred to as "normal diet") for at least 3 weeks were prepared. Table 1 shows the composition of each diet.

**Table 1**

| Raw material name | Unit | Normal diet | Low-protein diet |
|---|---|---|---|
| Casein | % (w/w) | 20 | 2.5 |
| L-Methionine | | 0.32 | 0.04 |
| β-Cornstarch | | 33.45 | 34.15 |
| α-Cornstarch | | 10 | 22 |
| Sucrose | | 21.73 | 26.81 |
| Corn oil | | 5 | 5 |
| Cellulose powder | | 5 | 5 |
| AIN-93G mineral mix | | 3.5 | 3.5 |
| AIN-93 vitamin mix | | 1 | 1 |
| Total | | 100 | 100 |

Each group had access to tap water ad libitum for at least 3 weeks. After fasting with ad libitum access to water for 24 hours, jugular vein catheterization was performed under isoflurane anesthesia, and the other end of the catheter was passed under the skin, exited from the back, and was connected to a syringe for an administration liquid via a harness. The rats were then housed in metabolic cages. In addition, after the catheterization, an antibiotic (ampicillin) was injected intramuscularly at a dose of 5 mg/100 µE/rat.

After recovery from anesthesia, blood (400 µL) was collected from the catheter placed in the jugular vein. The animals in each group, which were grouped so that the body weights and the plasma phosphorus concentrations were equal between groups, were refed by continuously infusing an intravenous nutritional infusion formulation having the composition shown in Table 2 (hereinafter referred to as "the infusion formulation of Comparative Example 1," which contains amino acids in a total amount of 3.6 g/100 kcal) over a period of 16 hours so that the infusion formulation of Comparative Example 1 was administered at a calorific value of 150 kcal per kg body weight.

The intravenous nutritional infusion formulation having the composition shown in Table 2 was prepared by mixing the upper-chamber solution, small-chamber V solution, and small-chamber T solution shown in Table 3 and the lower-chamber solution shown in Table 4 before use.

Blood (400 µL) was also collected from the same line at the completion of administration. Plasma was separated from the blood obtained in the test, and the inorganic phosphorus concentration was measured by an enzymatic method (Determiner L, produced by Hitachi Chemical Diagnostics Systems Co., Ltd.).

**Table 2**

| Nutritional infusion (1000 mL) | | |
|---|---|---|
| Component | | Content |
| Saccharide | Glucose | 175 g |
| | Sugar concentration | 17.5% |
| Electrolyte | Na⁺ | 50 mEq |
| | K⁺ | 27 mEq |
| | Mg²⁺ | 5 mEq |
| | Ca²⁺ | 5 mEq |
| | Cl⁻ | 50 mEq |
| | SO₄² | 5 mEq |
| | Acetate | 48 mEq |
| | L-Lactate | 14 mEq |
| | Citrate³ | 12 mEq |
| | P | 6 mmol (187 mg) |
| Vitamin | Thiamine chloride hydrochloride | 3.84 mg |
| | Riboflavin sodium phosphate | 2.3 mg |
| | Pyridoxine hydrochloride | 3.675 mg |
| | Cyanocobalamin | 2.5 µg |
| | Nicotinamide | 20 mg |
| | Panthenol | 7 mg |
| | Folic acid | 0.3 mg |
| | Biotin | 30 µg |
| | Ascorbic acid | 100 mg |
| | Vitamin A oil | 1650 vitamin A unit |
| | Cholecalciferol | 2.5 µg |
| | Tocopherol acetate | 5 mg |
| | Phytonadione | 0.075 mg |
| Trace element | Iron (Fe) | 10 pmol |
| | Manganese (Mn) | 0.5 pmol |
| | Zinc (Zn) | 30 pmol |
| | Copper (Cu) | 2.5 pmol |
| | Iodine (I) | 0.5 pmol |
| Amino acid | Total free amino acid amount 20 g, 30 g, 40 g | 30 g |
| | Total nitrogen amount 3.13 g, 4.70 g, 6.27 g | 4.70 g |
| | Essential amino acid/nonessential amino acid | 1.44 |
| | Branched-chain amino acid content | 30 w/w% |
| Total calorific value | | 820 kcal |
| Non-protein calorific value | | 700 kcal |
| Non-protein calorific value/nitrogen | | 149 |

**Table 3**

| Upper-chamber solution (492 mL) | | |
|---|---|---|
| Component | | Content |
| Saccharide | Glucose | 175 g |
| Electrolyte | Sodium chloride | 2.050 g |
| | Potassium chloride | 0.746 g |
| | L-Sodium lactate | 1.590 g |
| | Potassium dihydrogen phosphate | 0.821 g |
| Trace element | Potassium iodide | 0.0830 mg |
| Vitamin | Thiamine chloride hydrochloride (on a thiamine basis) | 3.84 mg (3.0 mg) |
| | Pyridoxine hydrochloride (on a pyridoxine basis) | 3.675 mg (3.0 mg) |
| | Cyanocobalamin | 2.5 µg |
| | Panthenol (on a pantothenic acid basis) | 7 mg (7.5 mg) |
| Additive | Glacial acetic acid (pH adjuster) | Appropriate amount |

| Small-chamber V solution (4 mL) | | |
|---|---|---|
| Component | | Content |
| Vitamin | Riboflavin sodium phosphate (on a riboflavin basis) | 2.3 mg (1.8 mg) |
| | Ascorbic acid | 100 mg |
| | Biotin | 30 µg |
| | Vitamin A oil | 1650 vitamin A unit |
| | Cholecalciferol | 2.5 µg |
| | Tocopherol acetate | 5 mg |
| | Phytonadione | 0.075 mg |
| Additive | Polysorbate 80 | 22.8 mg |
| | Polysorbate 20 | 4 mg |
| | Macrogol 400 | 40 mg |
| | Sodium hydroxide (pH adjuster) | Appropriate amount |
| | Sodium dihydrogen phosphate hydrate (pH adjuster) | Appropriate amount |

| Small-chamber T solution (4 mL) | | |
|---|---|---|
| Component | | Content |
| Trace element | Ferric chloride hydrate | 2.703 mg |
| | Manganese chloride hydrate | 0.09895 mg |
| | Zinc sulfate hydrate | 8.625 mg |
| | Copper sulfate hydrate | 0.624 mg |
| Additive | Sodium chondroitin sulfate | 2.793 mg |
| | Sodium hydroxide (pH adjuster) | Appropriate amount |

**Table 4**

| Lower-chamber solution (500 mL) | | |
|---|---|---|
| Component | | Content |
| Amino acid | L-Leucine | 4.20 g |
| | L-Isoleucine | 2.40 g |
| | L-Valine | 2.40 g |
| | L-Lysine acetate (on an L-lysine basis) | 4.44 g (3.15 g) |
| | L-Threonine | 1.71 g |
| | L-Tryptophan | 0.60 g |
| | L-Methionine | 1.17 g |
| | Acetylcysteine (on an L-cysteine basis) | 0.40 g (0.30 g) |
| | L-Phenylalanine | 2.10 g |
| | L-Tyrosine | 0.15 g |
| | L-Arginine | 3.15 g |
| | L-Histidine | 1.50 g |
| | L-Alanine | 2.40 g |
| | L-Proline | 1.50 g |
| | L-Serine | 0.90 g |
| | Glycine | 1.77 g |
| | L-Aspartic acid | 0.30 g |
| | L-Glutamic acid | 0.30 g |
| Electrolyte | Calcium chloride hydrate | 0.370 g |
| | Magnesium sulfate hydrate | 0.620 g |
| | Potassium acetate | 1.080 g |
| Vitamin | Nicotinamide | 20 mg |
| | Folic acid | 0.3 mg |
| Additive | Sodium bisulfite | 15 mg |
| | Citric acid hydrate (pH adjuster) | Appropriate amount |

Fig. 1 and Table 5 show the results. "NPD (before)" indicates the plasma phosphorus concentration before refeeding with the infusion formulation of Comparative Example 1 in the rats given the normal diet, and "LPD (before)" indicates the plasma phosphorus concentration before refeeding with the infusion formulation of Comparative Example 1 in the rats given the low-protein diet. "NPD (refeeding)" indicates the plasma phosphorus concentration after refeeding with the infusion formulation of Comparative Example 1 in the rats given the normal diet, and "LPD (refeeding)" indicates the plasma phosphorus concentration after refeeding with the infusion formulation of Comparative Example 1 in the rats given the low-protein diet. In the present specification, the plasma inorganic phosphorus concentration values are expressed as mean ± standard deviation.

**Table 5**

| | LPD (before) | NPD (before) | LPD (refeeding) | NPD (refeeding) |
|---|---|---|---|---|
| Plasma inorganic phosphorus concentration (mg/dL) | 7.41 ± 0.91 | 6.99 ± 0.48 | 3.85 ± 0.50 | 5.71 ± 0.19 |

There was no significant difference only between the "LPD (before)" and "NPD (before)" groups; and for all of the other combinations, there were significant differences (n = 10, p<0.05, Tukey's multiple comparison).

Even when the rats that had been given the normal diet were refed with the infusion formulation of Comparative Example 1, the plasma phosphorus concentration decreased ("NPD (before)" versus "NPD (refeeding)"); however, a more pronounced decrease in the plasma phosphorus concentration was observed when the rats that had been given the low-protein diet were refed with the infusion formulation of Comparative Example 1 ("LPD (before)" versus "LPD (refeeding)").

The above results showed that hypophosphatemia did not develop solely due to the undernutrition state caused by ingestion of the low-protein diet and that hypophosphatemia developed when refeeding with the infusion formulation of Comparative Example 1 was further performed.

### 2. Relationship 1 Between Amino Acid Content and Hypophosphatemia at the Time of Refeeding with Nutritional Infusion Formulation

Male Wistar rats (7 weeks old at the time of purchase, Charles River Laboratories Japan, Inc.) were acclimated and given the low-protein diet for at least 3 weeks. The rats had access to tap water ad libitum for at least 3 weeks. After fasting with ad libitum access to water for 24 hours, jugular vein catheterization was performed under isoflurane anesthesia, and the other end of the catheter was passed under the skin, exited from the back, and was connected to a syringe for an administration liquid via a harness. The rats were then housed in metabolic cages. In addition, after the catheterization, an antibiotic (ampicillin) was injected intramuscularly at a dose of 5 mg/100 µL/rat.

After recovery from anesthesia, the rats were grouped so that the body weights and the plasma phosphorus concentrations were equal between groups. One group was refed by continuously infusing the infusion formulation of Comparative Example 1 over a period of 16 hours so that the infusion formulation was administered in an amount of 183 mL per kg body weight, and the other group was refed by continuously infusing an infusion formulation obtained by removing the amino acids from the infusion formulation of Comparative Example 1 (hereinafter referred to as "the infusion formulation of Example 1") over a period of 16 hours so that the infusion formulation was administered in an amount of 183 mL per kg body weight.

Table 6 shows the results. Symptoms of hypophosphatemia were suppressed in the rats in an undernutrition state when they were refed with the infusion formulation of Example 1, which contains no amino acids, compared with when they were refed with the infusion formulation of Comparative Example 1, which contains amino acids in a total amount of 3.6 g/100 kcal.

**Table 6**

| | Amino acid content per 100 kcal of infusion formulation used for refeeding | Plasma inorganic phosphorus concentration after refeeding (mg/dL) |
|---|---|---|
| Infusion formulation of Comparative Example 1 | 3.6 g | 4.17 ± 0.36 |
| Infusion formulation of Example 1 | 0 g | 6.64 ± 0.63 |

The above results suggested that the amino acid content is related to the suppression of symptoms of hypophosphatemia.

### 3. Relationship 2 Between Amino Acid Content and Hypophosphatemia at the Time of Refeeding with Nutritional Infusion Formulation

To confirm whether the suppression of hypophosphatemia depends on the dose of amino acids at the time of refeeding, the following experiment was performed.

Male Wistar rats (7 weeks old at the time of purchase, Charles River Laboratories Japan, Inc.) were acclimated and given the low-protein diet for at least 3 weeks. The rats had access to tap water ad libitum for at least 3 weeks. After fasting with ad libitum access to water for 24 hours, jugular vein catheterization was performed under isoflurane anesthesia, and the other end of the catheter was passed under the skin, exited from the back, and was connected to a syringe for an administration liquid via a harness. The rats were then housed in metabolic cages. In addition, after the catheterization, an antibiotic (ampicillin) was injected intramuscularly at a dose of 5 mg/100 µE/rat.

After recovery from anesthesia, the rats were grouped so that the body weights and the plasma phosphorus concentrations were equal between groups, and refed by continuously infusing the infusion formulations shown in Table 7 over a period of 16 hours so that each formulation was administered at a calorific value of 150 kcal per kg body weight.

The amount of the amino acids in the infusion formulation of Example 3 was one-third of the amount of the amino acids in the infusion formulation of Comparative Example 1, i.e., 1.2 g/100 kcal. The amount of the amino acids in the infusion formulation of Example 4 was two-thirds of the amount of the amino acids in the infusion formulation of Comparative Example 1, i.e., 2.4 g/100 kcal. In the infusion formulations of Example 2, Example 3, and Example 4, instead of the reduced amounts of amino acids, the amounts of glucose were increased until the total amounts of glucose shown in Table 7 were reached so that the total calorific value was 820 kcal/L (1000 mL).

**Table 7**

| | Content and total calorific value per 1000 mL of each infusion formulation | | | Amino acid concentration | Amino acid content per 100 kcal |
|---|---|---|---|---|---|
| | Amino acid | Glucose | Total calorific value | | |
| Infusion formulation of Example 2 | 0 g | 205 g | 820 kcal | 0% (w/v) | 0 g |
| Infusion formulation of Example 3 | 10 g | 195 g | | 1% (w/v) | 1.2 g |
| Infusion formulation of Example 4 | 20 g | 185 g | | 2% (w/v) | 2.4 g |
| Infusion formulation of Comparative Example 1 | 30 g | 175 g | | 3% (w/v) | 3.6 g |

Fig. 2 and Table 8 show the results. In Fig. 2, "0%" indicates the infusion formulation of Example 2 (containing no amino acids (containing 0 g/100 kcal)), "1%" indicates the infusion formulation of Example 3 (containing amino acids in a total amount of 1.2 g/100 kcal), "2%" indicates the infusion formulation of Example 4 (containing amino acids in a total amount of 2.4 g/100 kcal), and "3%" indicates the infusion formulation of Comparative Example 1 (containing amino acids in a total amount of 3.6 g/100 kcal).

There were significant differences for all of the combinations (n = 8/group, p<0.05, Tukey's multiple comparison).

**Table 8**

| | Plasma inorganic phosphorus concentration (mg/dL) |
|---|---|
| Infusion formulation of Example 2 | 6.00 ± 0.53 |
| Infusion formulation of Example 3 | 5.11 ± 0.43 |
| Infusion formulation of Example 4 | 4.19 ± 0.57 |
| Infusion formulation of Comparative Example 1 | 3.30 ± 0.36 |

The infusion formulations with a lower amino acid content per 100 kcal achieved suppression of hypophosphatemia to a greater extent. These results revealed that suppression of symptoms of hypophosphatemia caused by refeeding rats in an undernutrition state depends on the dose of amino acids in the total calorific value at the time of refeeding.

### Enteral Nutritional Formulation

### 4. Relationship Between Amino Acid Content and Hypophosphatemia at the Time of Refeeding with Enteral Nutritional Formulation

Male Wistar rats (8 weeks old at the time of purchase, Charles River Laboratories Japan, Inc.) were acclimated, and then a group given the normal diet for 3 weeks and a group given the low-protein diet for 3 weeks were prepared. The rats had access to tap water ad libitum.

The enteral nutritional formulations (the liquid food of Example 5, the liquid food of Example 6, and the liquid food of Comparative Example 2) shown in Table 9 were used for refeeding.

**Table 9**

| | Calorific value (kcal/100 mL) | Protein (g/100 kcal) | Lipid (g/100 kcal) | Saccharide (g/100 kcal) |
|---|---|---|---|---|
| Liquid food of Comparative Example 2 | 100 | 6 | 4.2 | 9.5 |
| Liquid food of Example 5 | 100 | 3 | 4.9 | 11 |
| Liquid food of Example 6 | 100 | 0 | 5.6 | 12.5 |

The liquid food of Comparative Example 2 contains 6 g/100 kcal of whey peptides (Aria Foods Ingredients) as protein, and the calorific percentages of protein, lipid, and saccharide were 24%, 38%, and 38%, respectively. In addition, the minerals and vitamins shown in Table 10 were added, and as dietary fibers, 0.4 g of gum ghatti and 0.067 g of a cellulose preparation were added per 100 kcal. As lipids, vegetable oil and medium-chain fatty acid triglyceride were used at a ratio of 1:1. As a saccharide, maltodextrin was used.

In the liquid food of Example 5 (containing 3 g/100 kcal of protein) and the liquid food of Example 6 (containing no protein (0 g/100 kcal)), the calorific concentration corresponding to the reduction in protein from the liquid food of Comparative Example 2 was compensated for by the lipids and saccharide so that the lipid:saccharide ratio was always 1:1.

**Table 10**

| Component | | Unit | Content (/100 kcal) |
|---|---|---|---|
| Mineral etc. | Sodium | mg | 61.4 |
| | Potassium | mg | 188 |
| | Magnesium | mg | 26.9 |
| | Calcium | mg | 112 |
| | Phosphorus | mg | 57.3 |
| | Chromium | µg | 2.57 |
| | Molybdenum | µg | 4.75 |
| | Manganese | mg | 0.336 |
| | Iron | mg | 0.633 |
| | Copper | mg | 0.0829 |
| | Zinc | mg | 1.45 |
| | Selenium | µg | 6.38 |
| | Iodine | µg | 13.75 |
| Vitamin | Vitamin B₁ | mg | 0.225 |
| | Vitamin B₂ | mg | 0.2375 |
| | Niacin | mgNE | 2.25 |
| | Vitamin B₆ | mg | 0.3 |
| | Folic acid | µg | 30 |
| | Vitamin B₁₂ | mg | 0.3 |
| | Biotin | µgRE | 4.25 |
| | Pantothenic acid | mg | 1.25 |
| | Vitamin C | mg | 52.5 |
| | Vitamin A | µg | 67.5 |
| | Vitamin E | mg | 2.375 |
| | Vitamin D | µg | 1.25 |
| | Vitamin K | µg | 6.25 |

These liquid foods were prepared by the following method.

The components to be incorporated were added to water, followed by mixing with a mixer. Thereafter, the mixtures were homogenized (50 MPa, 2 passes) with a high-pressure homogenizer (LAB-1000, APV). The resulting homogenized liquid foods (oil-in-water emulsion compositions) were poured in pouches and sterilized with heat (121°C, 10 minutes).

Before refeeding, blood was collected from the jugular veins of the rats given the normal diet and the rats given the low-protein diet, and the phosphorus concentrations in plasma were quantified colorimetrically in a clinical chemistry analyzer (FUJI DRI-CHEM SLIDE IP-P, produced by FUJIFILM Medical Co., Ltd.). The rats were grouped so that there was no variation in the values between groups.

After grouping, each liquid food shown in Table 9 was continuously administered through a gastric fistula created using a syringe pump over a period of 16 hours so that the total calorific amount administered was 210 kcal per kg body weight. After the completion of administration, blood was collected from the jugular veins of the animals to which the test substances were administered, plasma was separated, and the concentration of inorganic phosphorus in plasma was measured by an enzymatic method (Determiner L, produced by Hitachi Chemical Diagnostics Systems Co., Ltd.).

Fig. 3 and Table 11 show the results. In Fig. 3, "20P-HP" indicates a group that received the normal diet before refeeding and the liquid food of Comparative Example 2 (containing 6 g/100 kcal of protein) at the time of refeeding; "20P-MP" indicates a group that received the normal diet before refeeding and the liquid food of Example 5 (containing 3 g/100 kcal of protein) at the time of refeeding; and "20P-LP" indicates a group that received the normal diet before refeeding and the liquid food of Example 6 (containing 0 g/100 kcal of protein) at the time of refeeding.

"LDP-HP" indicates a group that received the low-protein diet before refeeding and the liquid food of Comparative Example 2 (containing 6 g/100 kcal of protein) at the time of refeeding; "LDP-MP" indicates a group that received the low-protein diet before refeeding and the liquid food of Example 5 (containing 3 g/100 kcal of protein) at the time of refeeding; and "LDP-LP" indicates a group that received the low-protein diet before refeeding and the liquid food of Example 6 (containing 0 g/100 kcal of protein) at the time of refeeding.

**Table 11**

| | Plasma inorganic phosphorus concentration (mg/dL) after refeeding | | |
|---|---|---|---|
| | Refeeding with liquid food of Comparative Example 2 (6 g/100 kcal) | Refeeding with liquid food of Example 5 (3 g/100 kcal) | Refeeding with liquid food of Example 6 (0 g/100 kcal) |
| Ingestion of normal diet before refeeding | 5.0 ± 0.5 | 5.1 ± 0.4 | 5.4 ± 0.5 |
| Ingestion of low-protein diet before refeeding | 2.6 ± 0.7 | 4.3 ± 1.2 | 5.6 ± 0.7 |

In the rats given the normal diet before refeeding, no significant differences in the blood phosphorus concentration were observed when refeeding was performed using any of the liquid foods having protein concentrations of 6, 3, and 0 g/100 kcal.

In contrast, in the undernourished rats given the low-protein diet before refeeding, as the protein concentration of the total calorific value in the liquid food at the time of refeeding was lower, hypophosphatemia was suppressed to a greater extent (significant differences between the groups in all of the combinations, Tukey's multiple comparison, n = 7 to 8, p<0.05).

## Claims

1. A nutritional formulation for use in suppressing a symptom of refeeding syndrome, the nutritional formulation comprising a protein and/or an amino acid in a total amount of 3.5 g or less per 100 kcal.

2. The nutritional formulation according to claim 1, for use in suppressing a symptom of hypophosphatemia.

3. The nutritional formulation according to claim 1 or 2, for use in administration to a patient in an undernutrition state.

4. The nutritional formulation according to any one of claims 1 to 3, which is an intravenous nutritional infusion formulation.

5. The intravenous nutritional infusion formulation according to claim 4, further comprising a sugar.

6. The nutritional formulation according to any one of claims 1 to 3, which is an enteral nutritional formulation.

7. The enteral nutritional formulation according to claim 6, further comprising a saccharide.

8. A method for providing nutritional support for a patient in an undernutrition state using a nutritional formulation, wherein the nutritional formulation comprises 0 to 3.5 g of a protein or an amino acid per 100 kcal.
